# EUROPÄISCHE PATENTANMELDUNG

(11) **EP 4 389 046 A1**
(43) Veröffentlichungstag der Anmeldung: **26.06.2024**
(21) Anmeldenummer: 23216971.4
(22) Anmeldetag: 15.12.2023
(51) Int. Cl.: A61B 34/00, A61B 46/10, A61B 17/00

(54) **CHIRURGISCHES INSTRUMENT**

(30) Priorität: 20.12.2022 DE 102022134205
(71) Anmelder: Karl Storz SE & Co. KG, 78532 Tuttlingen (DE)
(72) Erfinder: Grüner, Sven, 78532 Tuttlingen (DE); Stefan, Jochen, 78532 Tuttlingen (DE)

(57) **Zusammenfassung**

Die vorliegende Erfindung betrifft ein chirurgisches Instrument (10) mit einem Gehäuse (12), das am proximalen Ende (14) des chirurgischen Instruments (10) angeordnet ist, einer an dem Gehäuse (12) angeordneten Schnittstelle (16) zur Kopplung mit einer Antriebseinheit (122), einem in dem Gehäuse (12) angeordneten Lenkgetriebe (18), wobei das Lenkgetriebe (18) eine räumlich ausrichtbare Taumelscheibe (20) aufweist, und wenigstens einem Spindeltrieb (22, 24) zum Antreiben (22, 24) des Lenkgetriebes (18). Die vorliegende Erfindung betrifft ferner ein steriles Antriebssystem (120) für ein solches chirurgisches Instrument (10) und ein Verfahren für die sterile Montage eines Antriebssystems (120).

## Beschreibung

Die vorliegende Erfindung betrifft ein chirurgisches Instrument. Ferner betrifft die vorliegende Erfindung ein steriles Antriebssystem für ein solches chirurgisches Instrument. Die vorliegende Erfindung betrifft auch ein Verfahren zur Montage eines solchen sterilen Antriebssystems.

Aus dem Stand der Technik sind chirurgische Instrumente bekannt, die manuell oder von einem Roboter geführt werden können und die Werkzeuge aufweisen, deren Werkzeugspitze mittels mehrerer ineinandergreifender Schwenkglieder verschwenkt werden kann. Diese Schwenkglieder sind mit einer Vielzahl von Lenkdrähten oder Lenkseilen verbunden, um eine feinfühlige Steuerung der Werkzeugspitze zu erreichen. Mit den Lenkdrähten kann eine gleichmäßige Kraftverteilung in allen Abwicklungsrichtungen erreicht werden.

US 10,485,621 B2 offenbart eine an einem Roboter anbringbare Antriebseinheit, die eine Schnittstellenfläche zur Kopplung mit einem chirurgischen Instrument aufweist. An der Schnittstellenfläche sind verschiedene mechanische und elektrische Schnittstellen zum Übertragen von mechanischen Bewegungen und elektrischen Signalen angeordnet. Ferner offenbart US 10,485,621 B2 einen Überzug für einen Roboterarm, der eine Öffnung für die Antriebseinheit und das mit der Antriebseinheit koppelbare chirurgische Instrument aufweist.

Aus DE 10 2019 121 092 A1 ist ein chirurgisches Instrument bekannt, das ein Lenkgetriebe aufweist. Mit dem Lenkgetriebe können die Stellwinkel von zwei Antrieben direkt auf eine räumlich verstellbare Scheibe (Taumelscheibe) übertragen werden, um diese zur Steuerung der Werkzeugspitze auszurichten. Dazu werden an der Taumelscheibe Lenkdrähte befestigt, sodass sich die Werkzeugspitze durch Ausrichtung der Taumelscheibe stufenlos und flüssig steuern lässt. Auch aus US 10,105,128 B2 ist ein Getriebe für ein chirurgisches Instrument bekannt. Das Getriebe dieses chirurgischen Instruments umfasst eine verlagerbare Scheibe und Gelenksstangen, die zum Bewegen der verlagerbaren Scheibe angeordnet sind.

Ausgehend von diesem Stand der Technik ist es eine Aufgabe der vorliegenden Erfindung, ein chirurgisches Instrument bereitzustellen, das mit einer an einem Roboterarm anbringbaren Antriebseinheit derart koppelbar ist, dass das chirurgische Instrument bei einem intraoperativen Wechsel steril bleibt.

Diese Aufgabe wird mit einem chirurgischen Instrument gemäß dem Patentanspruch 1 gelöst. Weitere Ausführungsformen sind in den abhängigen Ansprüchen angegeben.

Das erfindungsgemäße chirurgische Instrument umfasst ein Gehäuse, das am proximalen Ende des chirurgischen Instruments angeordnet ist, eine an dem Gehäuse angeordnete Schnittstelle zur Kopplung mit einer Antriebseinheit, ein in dem Gehäuse angeordnetes Lenkgetriebe, wobei das Lenkgetriebe eine räumlich ausrichtbare Taumelscheibe aufweist, und wenigstens einen Spindeltrieb zum Antreiben des Lenkgetriebes.

Mit dem eine Taumelscheibe aufweisenden Lenkgetriebe kann ein proximal mit einer roboterseitigen Antriebseinheit koppelbares chirurgisches Instrument bereitgestellt werden, das einen sterilen intraoperativen Wechsel der chirurgischen Instrumente ermöglicht.

Die Schnittstelle kann an einer senkrecht zur Längsachse eines Schafts verlaufenden Kopplungsfläche des Gehäuses angeordnet sein, wobei sich der Schaft durch die Kopplungsfläche erstreckt. Mit anderen Worten erstreckt sich der Schaft durch die Kopplungsfläche und verläuft senkrecht zu dieser Kopplungsfläche. Ausgehend von dem am proximalen Ende des chirurgischen Instruments angeordneten Gehäuse erstreckt sich der Schaft in Richtung des distalen Endes des chirurgischen Instruments, an dem das eigentliche Werkzeug des chirurgischen Instruments angeordnet ist.

Die Schnittstelle kann Antriebsscheiben umfassen, die das Lenkgetriebe zum Verschwenken der Taumelscheibe antreiben und damit die eingangs erwähnten Schwenkglieder verschwenken können. Die Antriebsscheiben können an der Kopplungsfläche angeordnet sein. Die Antriebsscheiben können mit Motortreibscheiben an der roboterseitigen Antriebseinheit gekoppelt werden. Zumindest an den Motortreibscheiben kann eine Steriladapterplatte vorgesehen sein, um die unsterilen Antriebe abzudecken. Die Steriladapterplatte kann drehbare Elemente aufweisen, die eine drehmomentübertragende Kopplung zwischen den Antriebsscheiben an der Schnittstelle des chirurgischen Instruments und den Motortreibscheiben ermöglichen.

Das chirurgische Instrument kann zumindest einen ersten Spindeltrieb und einen zweiten Spindeltrieb aufweisen. Die Drehachsen des ersten Spindeltriebs und des zweiten Spindeltriebs können zueinander und zur Längsachse des chirurgischen Instruments parallel verlaufen. Das chirurgische Instrument kann in Richtung seiner Längsachse mit der Antriebseinheit gekoppelt werden. Dies bedeutet, dass die Kopplungsrichtung zur Kopplung des chirurgischen Instruments mit der Antriebseinheit parallel zur Längsachse und damit parallel zu den Drehachsen der Spindeltriebe verläuft.

Der erste Spindeltrieb und der zweite Spindeltrieb können jeweils eine Antriebsscheibe aufweisen. Die Antriebsscheiben können an der Kopplungsfläche angeordnet sein. Mit anderen Worten sind die Antriebsscheiben an der Schnittstelle angeordnet.

Das Lenkgetriebe kann einen ersten Schieber und einen zweiten Schieber aufweisen. Der erste Schieber kann dem ersten Spindeltrieb und der zweite Schieber dem zweiten Spindeltrieb zugeordnet sein. Der erste Schieber und der zweite Schieber können über die ihnen zugeordneten Spindeltriebe entlang der Drehachse des jeweiligen Spindeltrieb bewegt werden. Der erste Schieber und der zweite Schieber können über die ihnen zugeordneten Spindeltriebe entlang Längsachse des chirurgischen Instruments relativ zueinander bewegt werden. Mit anderen Worten können die Schieber ihre Relativpositionen relativ zueinander verändern.

Das Lenkgetriebe kann eine Klammer aufweisen. Die Klammer kann an der Taumelscheibe angeordnet sein. Die Klammer kann mit dem ersten Schieber und dem zweiten Schieber gekoppelt sein. Der erste Schieber und der zweite Schieber können durch Veränderungen ihrer Relativpositionen zueinander die Klammer bewegen und damit die Taumelscheibe zur Ausführung von bestimmten Bewegungsmustern antreiben.

Der erste Schieber und der zweite Schieber können jeweils einen Kugelkopf aufweisen. Die Klammer kann wenigstens zwei Kugelpfannen aufweisen. Die Kugelköpfe an den Schiebern können jeweils in eine der Kugelpfannen an der Klammer eingreifen und so die Klammer mit den Schiebern koppeln. Durch die Veränderung der Relativpositionen in axialer Richtung zwischen dem ersten Schieber und dem zweiten Schieber kann sich der Abstand zwischen den Kugelköpfen an dem ersten Schieber und dem zweiten Schieber verändern. Durch die Veränderungen der Abstände des Kugelkopfs an dem ersten Schieber und des Kugelkopfs an dem zweiten Schieber kann die Klammer bewegt werden. Die Kugelköpfe der Schieber und die Kugelpfannen an der Klammer können eine gelenkige Kopplung der Klammer mit den Schiebern bereitstellen, sodass die Klammer gelenkige Bewegungen ausführen kann.

Die Klammer kann eine Klammerbasis und zwei Klammerarme aufweisen. Die Klammerarme können jeweils einen Endabschnitt aufweisen, der von einem freien Ende der Klammerarme gebildet wird. Jeweils eine der Kugelpfannen kann an einem Endabschnitt eines Klammerarms angeordnet sein. Die Kugelpfannen an den Endabschnitten der Klammerarme können mit den Kugelköpfen an den Schiebern gekoppelt werden.

Die Klammerarme können zwischen ihren Endabschnitten eine Klammeröffnung festlegen. Die Klammeröffnung kann der Klammerbasis gegenüberliegen. Über die Klammeröffnung kann die Klammer mit der Taumelscheibe in Eingriff gebracht werden. Durch die Verbindung der Klammer mit der Taumelscheibe können die von den Schiebern erzeugten Bewegungsmuster von der Klammer auf die Taumelscheibe und damit auf die Schwenkglieder des Werkzeugs des chirurgischen Instruments übertragen werden.

Die Klammer kann zumindest abschnittsweise elastisch ausgebildet sein. Dadurch kann die Klammer Verformungen elastisch aufnehmen, die aus den axialen Bewegungen des ersten Schiebers und des zweiten Schiebers und den sich damit verändernden Abständen zwischen den Kugelköpfen resultieren. Dies ermöglicht ferner die Verwendung von festeren Werkstoffen, die die hohen Drücke an den Kugelköpfen aufnehmen können.

Die Elastizität der Klammer kann zumindest über die geometrische Dicke und/oder den für die Klammer verwendeten Werkstoff eingestellt werden. Der Ausdruck "geometrische Dicke" kann sich in diesem Fall auf die radiale Dicke der Klammer beziehen. Wenn ein Abschnitt der Klammer dünner oder schlanker ausgeführt ist, kann die Klammer die voranstehend beschriebenen Verformungen aufnehmen, ohne dass die Klammer dabei plastisch und damit dauerhaft deformiert wird.

Die Klammer kann axiale Kontaktflächen für die Taumelscheibe aufweisen. Mit ihren axialen Kontaktflächen kann die Klammer sich zumindest abschnittsweise an die Taumelscheibe anlegen oder sich an der Taumelscheibe abstützen. Die axialen Kontaktflächen der Klammer können sich parallel zueinander erstrecken. Die axialen Kontaktflächen der Taumelscheibe sind zueinander entgegengesetzt an der Klammer ausgebildet. Mit anderen Worten können die axialen Kontaktflächen der Klammer voneinander wegweisen.

Die Taumelscheibe kann eine Nut aufweisen. Die Nut kann sich umlaufend um die Taumelscheibe herum erstrecken. Die Nut kann axiale Kontaktflächen für die Klammer aufweisen. Die axialen Kontaktflächen der Nut können sich im Wesentlichen parallel zueinander erstrecken. Die axialen Kontaktflächen der Nut können einander gegenüberliegend ausgebildet sein. Die Klammer kann in der Nut aufgenommen werden. Ist die Klammer in der Nut aufgenommen, können sich die axialen Kontaktflächen der Klammer und die axialen Kontaktflächen der Nut aneinander abstützen.

Die Klammer kann die Taumelscheibe zumindest abschnittsweise umschließen. Die Klammer kann die Taumelscheibe nahezu vollständig umschließen. Die Klammer kann mehr als 180°, insbesondere einen Winkelbereich zwischen 270° und 350° des Umfangs der Taumelscheibe umgeben.

Da die Klammer die Taumelscheibe nahezu vollständig umgeben kann, kann eine Umfangskontaktfläche zwischen der Klammer und der Taumelscheibe bereitgestellt werden. Diese relativ große Umfangskontaktfläche kann durch den Kontakt zwischen der Innenfläche der Klammer und dem Nutboden der Nut der Taumelfläche bereitgestellt werden. Die Innenseite der Klammer kann die Nutboden der Nut der Taumelscheibe kontaktieren. Durch die Umfangskontaktfläche zwischen der Klammer und der Taumelscheibe kann eine verbesserte Führung der Übertragung der Bewegungen von der Taumelscheibe erreicht und ein Verkippen oder Verkanten der Taumelscheibe verhindert werden.

Das Lenkgetriebe kann einen mit der Taumelscheibe gekoppelten Lenkring aufweisen. Der Lenkring kann die Taumelscheibe an deren Außenumfang umgeben.

Der erste Spindeltrieb und der zweite Spindeltrieb können jeweils mit einem Zahnrad zum Antreiben von wenigstens zwei Kegelrädern zusammenwirken. Die Kegelräder können zum Bewegen des Lenkrings drehmomentübertragend mit diesem gekoppelt werden. Die Kegelräder können jeweils über eine Lagerwelle an dem Gehäuse um eine Drehachse drehbar gelagert sein. Die Kegelräder können eine umlaufende bzw. vollständige Kegelverzahnung aufweisen. Die mit den Spindeltrieben zusammenwirkenden Zahnräder können an den Lagerwellen der Kegelräder drehbar gelagert sein.

Der Lenkring kann wenigstens zwei Kegelverzahnungsabschnitte aufweisen. Die Kegelverzahnungsabschnitte können segmentweise ausgebildet sein. Dies bedeutet, dass die Kegelverzahnungsabschnitte in Form von Kreissegmenten ausgebildet sein können. Die Kegelverzahnungsabschnitte können sich beispielswiese über einen Winkelbetrag über 60° bis 120° erstrecken. Die Kegelverzahnungsabschnitte können mit den Kegelrädern in Eingriff stehen, um den Lenkring bewegen zu können.

Die Kegelverzahnungsabschnitte können einstückig mit dem Lenkring ausgebildet sein. Die Kegelverzahnungsabschnitte können auch unabhängig von dem Lenkring als separates Bauteil oder separate Bauteile ausgebildet sein. In diesem Fall können die Kegelverzahnungsabschnitte mit dem Lenkring drehmomentübertragend verbunden werden. Mit einer zwei- oder mehrteiligen Ausführung des Lenkrings und der damit zu verbindenden Kegelverzahnungsabschnitte kann die Herstellung dieser Bauteile einfach gehalten werden.

Über die Zahnräder können die Kegelräder bewegt werden, um den Lenkring und die damit gekoppelte Taumelscheibe zu bewegen. Werden die Kegelräder entsprechend angetrieben, kann der Lenkring um die von den Lagerwellen gebildete Drehachse verschwenkt oder um eine zu dieser Drehachse senkrecht verlaufenden Drehachse verdreht werden.

Die vorliegende Erfindung betrifft ferner ein Antriebssystem für ein chirurgisches Instrument. Das Antriebssystem umfasst eine an einem Roboterarm anbringbare Antriebseinheit, eine an der Antriebseinheit angebrachte Steriladapterplatte, die eine Schnittstelle an der Antriebseinheit zumindest teilweise abdeckt und das chirurgische Instrument gemäß der voranstehend beschriebenen Art, wobei das chirurgische Instrument über die Schnittstelle mit der Antriebseinheit von proximal aus gekoppelt ist.

Die Steriladapterplatte kann so ausgebildet sein, dass sie drehbare Kupplungsscheiben aufweist, die eine drehmomentübertragende Kopplung zwischen den Antriebsscheiben an der Schnittstelle des chirurgischen Instruments und den unsterilen Motortreibscheiben die Antriebseinheit bereitstellt. Die drehbaren Kupplungsscheiben können eine Übertragung der Rotation von den unsterilen Motoren der Antriebseinheit auf das sterile chirurgische Instrument und insbesondere auf das Getriebe des chirurgischen Instruments ermöglichen.

Die Steriladapterplatte kann Teil eines Steriladapters sein, der die Antriebseinheit zumindest teilweise umgibt. Eine solcher Steriladapter kann mit der Steriladapterplatte auf die Antriebseinheit aufgesteckt werden.

Ferner kann zumindest die Steriladapterplatte mit einem sterilen Überzug verbunden sein, der zumindest einen Teil der Antriebseinheit umgibt. Der sterile Überzug kann beispielsweise von einer Folie gebildet werden.

Die vorliegende Erfindung betrifft ferner ein Verfahren für die sterile Montage eines Antriebssystems für ein chirurgisches Instrument, wobei das Antriebssystem eine an einem Roboterarm anbringbare Antriebseinheit, und das voranstehend beschriebene chirurgische Instrument umfasst, wobei das Verfahren die folgenden Schritte umfasst:
Anbringen einer Steriladapterplatte an der Antriebseinheit, die die Schnittstelle an der Antriebseinheit zumindest teilweise abdeckt, und
Anbringen des chirurgischen Instruments an der Schnittstelle der Antriebseinheit von proximal aus.

Auch im Zusammenhang mit dem Verfahren für die sterile Montage des Antriebssystems ist zu erwähnen, dass die Steriladapterplatte mit einem sterilen Überzug verbunden sein kann, der zumindest teilweise die Antriebseinheit umgibt. Der sterile Überzug kann auch teilweise eine Führungseinrichtung umgeben, über die die Antriebseinheit mit einem Roboterarm verbunden sein kann.

Die Anbringung der Steriladapterplatte an der Antriebseinheit kann den ersten Schritt darstellen. Die Steriladapterplatte deckt die unsterilen Motortreibscheiben der Antriebseinheit ab und hat drehbare Kupplungsscheiben, die eine Übertragung einer Rotation von den Motortreibscheiben auf die Antriebsscheiben des chirurgischen Instruments zulassen.

Im zweiten Schritt wird das chirurgische Instrument mit dem eine Taumelscheibe umfassenden Lenkgetriebe von proximal auf die Steriladapterplatte und damit auf die Antriebseinheit aufgesteckt. Diese Kopplung des chirurgischen Instruments mit der Antriebseinheit kann in Richtung der Längsachse des chirurgischen Instruments erfolgen. Diese Fügerichtung liegt auf einer Linie mit einem Trokar, sodass das chirurgische Instrument bei einer Operation anschließend in einer durchgehenden Bewegung in den Patienten hineingeschoben werden könnte.

Im Folgenden wird die Erfindung anhand von Figuren beispielhaft erläutert. Die Zeichnung, die Beschreibung und die Ansprüche enthalten zahlreiche Merkmale in Kombination. Der Fachmann wird die Merkmale zweckmäßigerweise auch einzeln betrachten und im Rahmen der Ansprüche sinnvoll in Kombination verwenden.

Falls von einem bestimmten Objekt mehr als ein Exemplar vorhanden ist, ist ggf. nur eines davon in den Figuren und in der Beschreibung mit einem Bezugszeichen versehen. Die Beschreibung dieses Exemplars kann entsprechend auf die anderen Exemplare von dem Objekt übertragen werden. Sind Objekte insbesondere mittels Zahlenwörtern, wie beispielsweise erstes, zweites, drittes Objekt etc. benannt, dienen diese der Benennung und/oder Zuordnung von Objekten. Demnach können beispielsweise ein erstes Objekt und ein drittes Objekt, jedoch kein zweites Objekt umfasst sein. Allerdings könnten anhand von Zahlenwörtern zusätzlich auch eine Anzahl und/oder eine Reihenfolge von Objekten ableitbar sein. #Die Zeichnungen, die Beschreibung und die Ansprüche enthalten zahlreiche Merkmale in Kombination. Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Es stellen dar:
- Figur 1: eine teilweise aufgebrochene perspektivische Ansicht eines chirurgischen Instruments gemäß einer ersten Ausführungsform und einer Antriebseinheit im gekoppelten Zustand;
- Figur 2: eine teilweise aufgebrochene perspektivische Ansicht des Lenkgetriebes des chirurgischen Instruments gemäß einer ersten Ausführungsform;
- Figuren 3 bis 5: verschiedene Ansichten einer Klammer zur Kopplung mit einer Taumelscheibe;
- Figur 6: eine perspektivische Ansicht eines chirurgischen Instruments gemäß einer zweiten Ausführungsform; und
- Figuren 7 und 8: perspektivische Ansichten eines Antriebssystems.

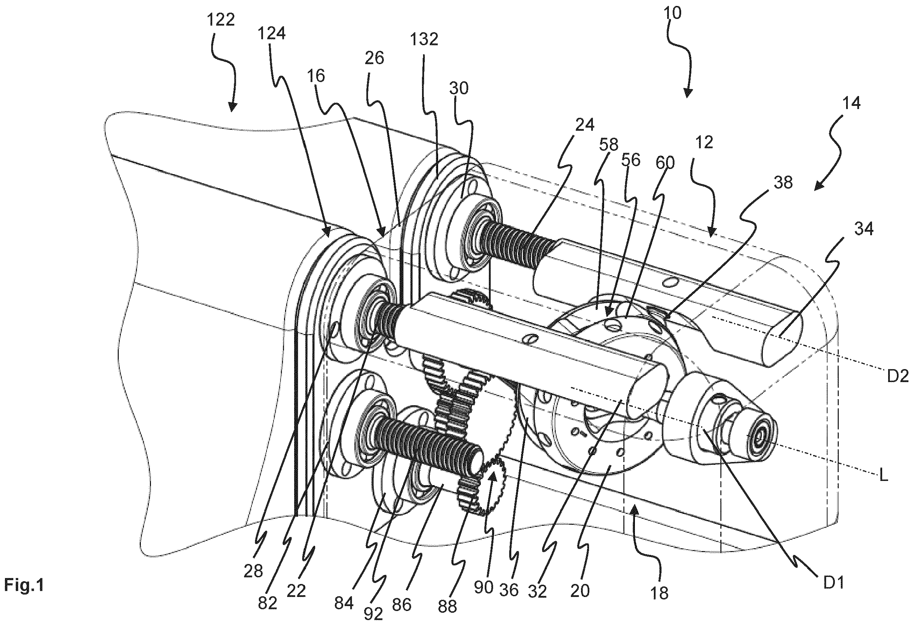

Figur 1 zeigt eine teilweise aufgebrochene perspektivische Ansicht eines chirurgischen Instruments 10 gemäß einer ersten Ausführungsform und einer Antriebseinheit 122 im gekoppelten Zustand.

Das chirurgische Instrument 10 umfasst ein Gehäuse 12. An dem Gehäuse 12 ist eine Schnittstelle 16 ausgebildet, die zur Kopplung mit der Antriebseinheit 122 dient. Die Schnittstelle 16 ist an einer senkrecht zur Längsachse L des chirurgischen Instruments 10 verlaufenden Kopplungsfläche 26 des Gehäuses 12 angeordnet. Die Längsachse L verläuft durch die Kopplungsfläche 26.

Die Schnittstelle 16 umfasst Antriebsscheiben 28 und 30, die an der Kopplungsfläche 26 angeordnet sind. Jeweils eine der Antriebsscheiben 28, 30 ist mit dem ersten Spindeltrieb 22 und dem zweiten Spindeltrieb 24 gekoppelt. Die Drehachsen D1 und D2 des ersten Spindeltriebs und des zweiten Spindeltriebs verlaufen parallel zueinander. Zudem verlaufen die Drehachsen D1 und D2 parallel zur Längsachse L.

Das chirurgische Instrument 10 weist ferner ein Lenkgetriebe 18 auf. Das Lenkgetriebe umfasst einen ersten Schieber 32 und einen zweiten Schieber 34. Der erste Schieber 32 ist dem ersten Spindeltrieb 22 und der zweite Schieber 34 dem zweiten Spindeltrieb 24 zugeordnet. Die Spindeltriebe 22 und 24 können die Schieber 32 und 34 entlang der Drehachsen D1 und D2 und damit auch parallel zu der Längsachse L verlagern. Jeder der Schieber 32 und 34 weist einen Kugelkopf 38 auf. Über die Kugelköpfe 38 sind die Schieber 32 und 34 mit einer Klammer 36 gekoppelt. Die Klammer 36 dient zur Übertragung von Bewegungen auf eine Taumelscheibe 20 des Lenkgetriebes 18.

Die Klammer 36 weist zwei Kugelpfannen 40 und 42 auf, die jeweils einem der Kugelköpfe 38 zugeordnet sind. Mit anderen Worten nimmt jeweils eine der Kugelpfannen 40, 42 einen Kugelkopf 38 an einem der Schieber 32, 34 auf. Die Kugelköpfe 38 und die Kugelpfannen 40, 42 koppeln die Klammer 36 gelenkig mit den Schiebern 32 und 34, sodass die Klammer 36 gelenkige Bewegungen ausführen kann.

Die Schieber 32 und 34 können über die Antriebsspindeln der Spindeltriebe 22 und 24 entlang der Längsachse L oder entlang der Drehachsen D1 und D2 relativ zueinander verlagert werden. Durch die Veränderungen der Relativpositionen der Schieber 32 und 34 relativ zueinander können sich die Abstände der Kugelköpfe 38 der Schieber 32 und 34 zueinander verändern. Durch die Änderungen der Relativpositionen der Schieber 32 und 34 und die dadurch erzeugten Änderungen der Abstände zwischen den Kugelköpfen 38 der Schieber 32 und 34 kann die Klammer 36 bewegt werden. Die Bewegungen der Klammer 36 können auf die Taumelscheibe 20 übertragen werden, die mit der Klammer 36 gekoppelt ist.

Gemäß Figur 1 ist die Klammer 36 in einer Nut 56 der Taumelscheibe 20 aufgenommen. Die Klammer 36 umgibt die Taumelscheibe 20 im Bereich der Nut 56 größtenteils. Anders ausgedrückt erstreckt sich die Klammer 36 über einen Großteil des Umfangs des Nutbodens (in Figur 1 nicht gezeigt) der Nut 56 der Taumelscheibe 20 und kann den Nutboden zumindest abschnittsweise kontaktieren.

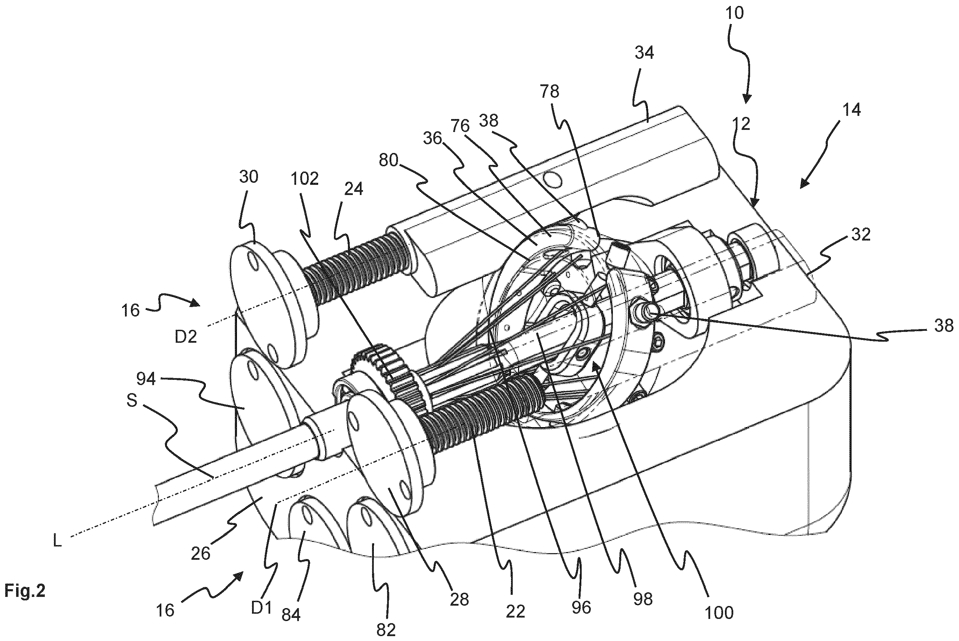

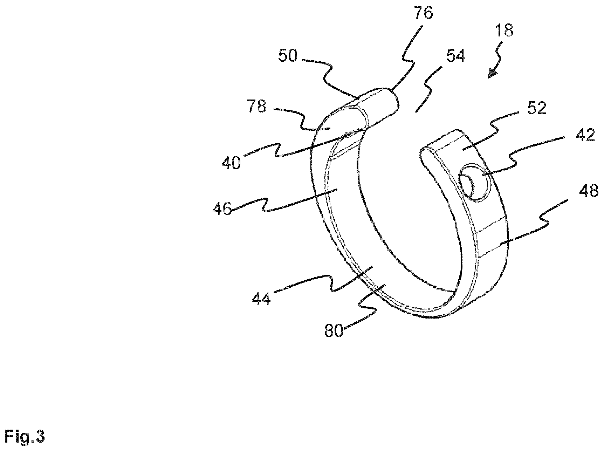

Die Nut 56 weist axiale Kontaktflächen 58 und 60 für die Taumelscheibe 20 auf. Die axialen Kontaktflächen 58 und 60 liegen einander gegenüber und erstrecken sich parallel zueinander. Die Klammer 36 weist ebenfalls axiale Kontaktflächen 76 und 78, die in Figur 2 gezeigt sind. Die Kontaktflächen 76 und 78 der Klammer 36 sind einander entgegengesetzt ausgebildet. Die Kontaktflächen 76 und 78 erstrecken sich im Wesentlichen parallel zueinander. Die Klammer 36 kann sich mit ihren axialen Kontaktflächen 76 und 78 an den axialen Kontaktflächen 58 und 60 der Nut 56 der Taumelscheibe 20 abstützen. Durch die Anlage der Kontaktflächen 58 und 60 der Nut 56 der Taumelscheibe 20 an den Kontaktflächen 76 und 78 der Klammer 36 kann ein Verkanten oder Verkippen der Taumelscheibe 20 bei der Übertragung von Bewegungen auf die Taumelscheibe 20 verhindert werden. Ein Verkanten oder Verkippen der Taumelscheibe 20 wird auch dadurch verhindert, dass die Klammer 36 den Nutboden der Nut 56 der Taumelscheibe 20 größtenteils umgibt. Die Klammer 36 weist eine Innenfläche 80 (siehe Figur 3), die sich an dem Nutboden anlegen kann. Die Innenfläche 80 bildet somit eine relativ große Umfangskontaktfläche. Durch den großflächigen Kontakt zwischen der Klammer 36 und dem Nutboden der Nut 56 der Taumelscheibe 20 kann die Führung der Taumelscheibe 20 durch die Klammer 36 verbessert und ein Verkippen oder ein Verkanten der Taumelscheibe 20 nahezu ausgeschlossen werden.

Die Schnittstelle 16 umfasst weitere Antriebsscheiben 82 und 84, die zum Antreiben weiterer Elemente des Lenkgetriebes vorgesehen sind. So kann beispielsweise die Antriebsscheibe 84 über eine Welle 86 und ein Stirnrad 88 ein Zahnradgetriebe 90 antreiben. Das Zahnradgetriebe 90 kann beispielsweise dazu dienen, den Schaft S in Rotation zu versetzen. Die Antriebsscheibe 82 ist mit einer weiteren Antriebsspindel 92 verbunden, die verschiedene Zusatzfunktionen des chirurgischen Instruments antreiben kann.

Ferner ist in Figur 1 die Antriebseinheit 122 gezeigt, die eine Schnittstelle 124 aufweist. Die Antriebseinheit 132 ist über ihre Schnittstelle 124 mit der Schnittstelle 16 des chirurgischen Instruments 10 gekoppelt. Zwischen den Schnittstellen 124
die Antriebseinheit 104 und der Schnittstelle 16 des chirurgischen Instruments 10 ist eine Steriladapterplatte 132 vorgesehen.

Die Steriladapterplatte 132 deckt die unsterile Schnittstelle 124 der Antriebseinheit 122 und insbesondere die unsterilen Motortreibscheiben Antriebseinheit 122 ab. Die Steriladapterplatte 132 ist dazu ausgebildet, dass die Rotation der Motortreibscheiben (nicht gezeigt) auf die Antriebsscheiben 28, 30, 82 und 84 an der Schnittstelle 16 des chirurgischen Instruments 10 übertragen werden kann. Dazu weist die Steriladapterplatte 132 nicht gezeigte drehbare Kupplungsscheiben auf, die die beschriebene Übertragung der Rotation zulassen.

Die Steriladapterplatte 132 kann lösbar mit der Antriebseinheit 122 verbunden werden. Die Steriladapterplatte 132 kann beispielsweise über eine Klick- oder Rastverbindung an der Antriebseinheit 122 angebracht werden. Wenn die Steriladapterplatte 132 an der Antriebseinheit 122 angebracht ist, kann das chirurgische Instrument 10 von proximal aus mit der Antriebseinheit 122 über die Steriladapterplatte 132 gekoppelt werden.

Figur 2 zeigt eine teilweise aufgebrochene perspektivische Ansicht des chirurgischen Instruments 10. An der an dem Gehäuse 12 ausgebildeten Kopplungsfläche 26 der Schnittstelle 16 ist neben den Antriebsscheiben 28, 30, 82 und 84 eine weitere Antriebsscheibe 94 vorgesehen.

Die Antriebsscheiben 28 und 30 sind mit den Spindeltrieben 22 und 24 verbunden, die ihrerseits die Schieber 32 und 34 antreiben. Die Schieber 32 und 34 weisen jeweils einen Kugelkopf 38 auf, die die Schieber 32, 34 mit der Klammer 36 koppeln. Durch Veränderungen der axialen Relativpositionen der Schieber 32 und 34 relativ zueinander können sich die Abstände zwischen den Kugelköpfen 38 an den Schiebern 32 und 34 verändern, wodurch die Klammer 36 bewegt wird. Die Bewegungen der Klammer 36 werden auf die Taumelscheibe 20 übertragen. An der Taumelscheibe 20 sind Lenkdrähte 96 befestigt, die die Bewegungen der Taumelscheibe 20 auf die Schwenkglieder des Werkzeugs an dem chirurgischen Instruments 10 oder die Werkzeugspitze übertragen und eine feinfühlige Steuerung des Werkzeugs ermöglichen.

Das Lenkgetriebe 18 weist eine Schaftwelle 98 auf. Die Taumelscheibe 20 ist über ein Kreuzgelenk 100 mit der Schaftwelle 98 verbunden. Das Kreuzgelenk 100 ist radial innerhalb der Taumelscheibe 20 und damit auch radial innerhalb der Klammer 36 angeordnet. Da die voranstehend beschriebene Umfangskontaktfläche zwischen der Innenseite 80 der Klammer 36 und dem Nutboden der Nut 56 der Taumelscheibe 20 das Kreuzgelenk 100 und insbesondere dessen Zentrum umschließt, wird die voranstehend beschriebene verbesserte Führung erreicht und ein Verkippen oder Verkanten der Taumelscheibe 20 verhindert. Die Klammer 36 dient zur flächigen Verteilung der Lenkkräfte zum Lenken der Schwenkglieder bzw. der Werkzeugspitze des Werkzeugs an dem chirurgischen Instrument 10. An der Schaftwelle 98 ist ferner ein Zahnrad 102 angeordnet, über das der Schaft S in eine Rotation versetzt werden kann.

Figur 3 zeigt eine perspektivische Ansicht der Klammer 36. Die Klammer 36 weist eine Klammerbasis 44 und zwei Klammerarme 46 und 48 auf. Die Klammerarme 46 und 48 erstrecken sich ausgehend von der Klammerbasis 44. Die Klammer 36 weist ferner Kugelpfannen 40, 42 auf. Die Kugelpfannen 40, 42 dienen zur Aufnahme der an den Schiebern 32 und 34 angeordneten Kugelköpfen 38 (siehe Figuren 1 und 2). Die Kugelpfannen 40, 42 sind an den Klammerarmen 46 und 48 ausgebildet. Die Kugelpfannen 40, 42 sind an den Endabschnitten 50, 52 im Bereich der freien Enden der Klammerarme 46 und 48 ausgebildet. Die Kugelpfannen 46 und 48 sind als durchgängige Öffnungen in der Klammer 36 ausgebildet. Die Klammerarme 46 und 48 legen zwischen ihren Endabschnitten 50, 52 die Klammeröffnung 54 fest. Über die Klammeröffnung 54 kann die Taumelscheibe 20 in die Klammer 36 eingesetzt werden. Die Klammeröffnung 54 liegt der Klammerbasis 44 im Wesentlichen gegenüber. Die Klammer 36 weist die voranstehend bereits beschriebene axialen Kontaktflächen 78 und 80 auf, die einander entgegengesetzt an der Klammer 36 ausgebildet sind. Die Kontaktflächen 78 und 80 der Klammer 36 erstrecken sich parallel zueinander.

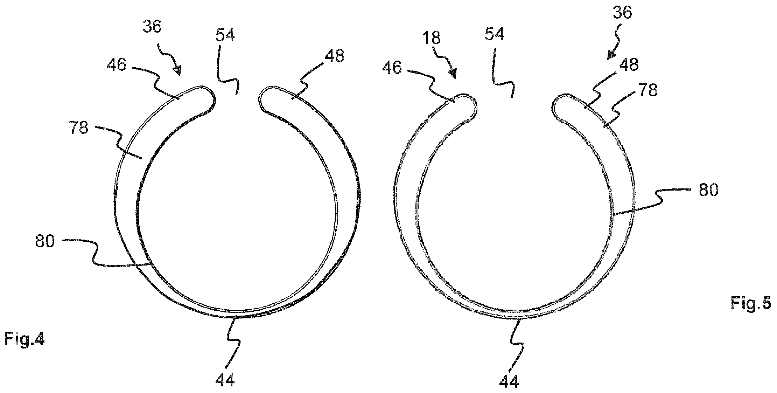

Die Figuren 4 und 5 zeigen zwei Ausführungsbeispiele der Klammer 36. In Figur 4 ist die Klammeröffnung 54 verglichen mit der Klammeröffnung 54 gemäß Figur 5 kleiner ausgeführt. Dies bedeutet, dass der Abstand zwischen den freien Enden der Klammerarme 46 und 48 in Figur 4 geringer ist als in Figur 5. Durch die Auswahl des Materials für die Klammer 36 und die geometrische Dicke der Klammer 36 in radialer Richtung können die elastischen Eigenschaften der Klammer 36 eingestellt werden. Wird beispielsweise die Klammer 36 oder zumindest ein Abschnitt der Klammer 36 schlanker bzw. in radialer Richtung dünner ausgeführt, kann die Klammer 36 die Verformung aufgrund der Veränderungen des Abstands zwischen den Kugelköpfen 38 an den Schiebern 32, 34 aufnehmen, ohne plastisch verformt zu werden.

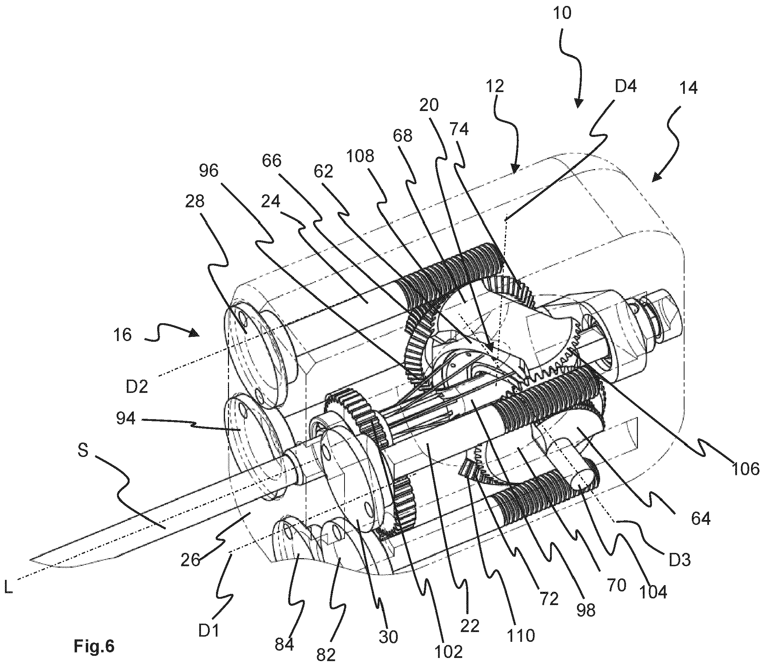

Figur 6 zeigt eine perspektivische Ansicht eines chirurgischen Instruments 10 gemäß einer zweiten Ausführungsform. Das chirurgische Instrument 10 weist das Gehäuse 12 und die Schnittstelle 16 auf. Die Schnittstelle 16 umfasst eine Kopplungsfläche 26, an der die Antriebsscheiben 28, 30, 82, 84 und 94 angeordnet sind. Die Kopplungsfläche 26 erstreckt sich senkrecht zur Längsachse L des chirurgischen Instruments 10. Ferner erstreckt sich die Kopplungsfläche 26 auch senkrecht zu den Drehachsen D1 und D2 des ersten Spindeltriebs 22 und des zweiten Spindeltriebs 24. Die Drehachsen D1 und D2 des ersten Spindeltriebs 22 und des zweiten Spindeltriebs 24 erstrecken sich parallel zueinander und parallel zur Längsachse L des chirurgischen Instruments 10.

Das chirurgische Instrument 10 kann einen Lenkring 62 aufweisen. Der Lenkring 62 ist mit der Taumelscheibe 20 gekoppelt. Die Taumelscheibe 20 ist innerhalb des Lenkrings 62 aufgenommen. Der Lenkring 62 kann über die Spindeltriebe 22 und 24 zur Ausführung von Bewegungen angetrieben werden. Die Spindeltriebe 22 und 24 treiben jeweils ein Zahnrad 64, 66 an. Die Zahnräder 64, 66 sind jeweils über eine Lagerwelle 104 an dem Gehäuse 12 gelagert. Die Lagerwellen 104 definieren eine dritte Drehachse D3 um die der Lenkring 18 verdreht werden kann. Die dritte Drehachse D3 erstreckt sich orthogonal zu den Drehachsen D1, D2 und zur Längsachse L des chirurgischen Instruments 10.

Die Zahnräder 64 und 66 sind mit Kegelrädern 66 und 70 gekoppelt. Die Kegelräder 66 und 70 weisen eine vollumfängliche Kegelverzahnung auf. Der Lenkring 62 ist mit zwei Kegelrädem 68 und 72 gekoppelt, die jedoch nur Abschnitte 106, 108, 112 mit einer Kegelverzahnung aufweisen. Mit anderen Worten sind die Kegelräder 68 und 72 nicht als vollständige Kegelräder ausgebildet, sondern nur mit den Kegelverzahnungsabschnitte 106, 108, 112 versehen. Der zweite Kegelverzahnungsabschnitte an dem Kegelrad 72 ist in Figur 6 nicht gezeigt. Die Kegelverzahnungsabschnitte 106 und 110 stehen mit dem Kegelrad 70 in Eingriff. Der Kegelverzahnungsabschnitte 108 und der nicht gezeigte Kegelverzahnungsabschnitte stehen mit dem Kegelrad 66 in Eingriff.

Der Lenkring 62 und die Kegelräder 68 und 72 sind so an dem Getriebegehäuse 12 gelagert, dass sie um eine vierte Drehachse D4 verdreht werden können. Die Bewegungen des Lenkrings 62 und damit der Taumelscheibe 20 können über die Drehrichtung der Spindeltriebe 22 und 24 gesteuert werden. Werden die Spindeltriebe 22 und 24 in gleicher Drehrichtung angetrieben, können der Lenkring 62 und die Taumelscheibe 20 um die dritte Drehachse D3 verschwenkt werden. Werden die Spindeltriebe 22 und 24 in gegenläufigen Drehrichtungen angetrieben, können die Kegelverzahnungsabschnitte 106, 108, 110, der Kegelrädern und die Taumelscheibe um die vierte Drehachse D4 verdreht werden. Diese beiden Bewegungen können zur Erzeugung von komplexen Bewegungsmustern überlagert werden.

Die Taumelscheibe 20 ist über die Lenkdrähte 96 mit den Schwenkglieder bzw. der Werkzeugspitze des an dem chirurgischen Instrument 10 angebrachten Werkzeug (nicht gezeigt) verbunden. Anders ausgedrückt kann das Werkzeug über die Lenkdrähte 96 gesteuert werden, die die von dem Lenkring 62 und der Taumelscheibe 20 erzeugten Bewegungen auf das Werkzeug übertragen. Die Schaftwelle 98 erstreckt sich durch die Taumelscheibe 20 und den Lenkring 62 durch. An der Schaftwelle 98 ist das Zahnrad 102 angeordnet, das den Schaft S in Rotation versetzen kann.

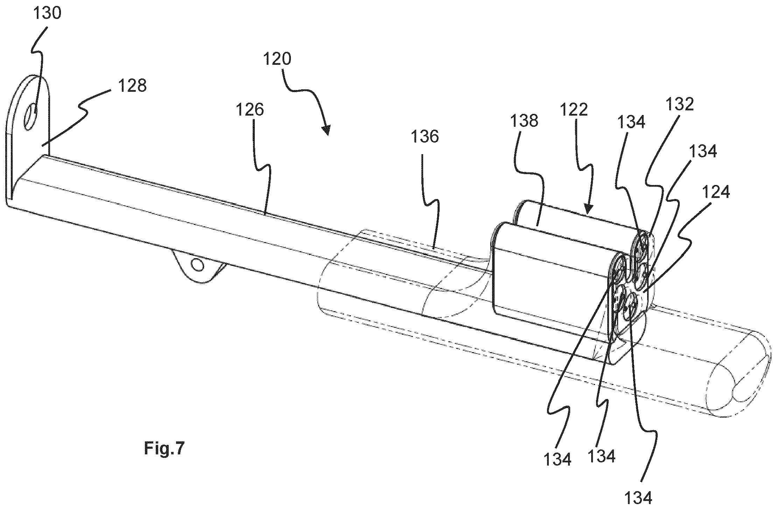

Figur 7 zeigt eine perspektivische Ansicht eines Antriebssystems 120. Das Antriebssystem 120 weist die Antriebseinheit 122 auf. Die Antriebseinheit 122 hat eine Schnittstelle 124, die zur Kopplung mit dem chirurgischen Instrument 10 ausgebildet ist. Die Antriebseinheit 122 ist an einer Führungseinrichtung 126 angeordnet, die zur Kopplung mit einem Roboterarm (nicht gezeigt) dient. Die Führungseinrichtung 126 weist einen Führungsvorsprung 128 mit einer Führungsöffnung 130 auf. In die Führungsöffnung 130 kann ein Trokar (nicht gezeigt) eingesetzt werden. Durch diese Führungsöffnung 130 kann der Schaft eines chirurgischen Instruments 10 geführt werden, wenn das chirurgische Instrument 10 mit der Antriebseinheit 122 gekoppelt wird. An der Schnittstelle 124 ist die Steriladapterplatte 132 angeordnet. Die Steriladapterplatte 132 ist lösbar an der Antriebseinheit 122 befestigt und deckt die unsterile Schnittstelle 124 und insbesondere die unsterilen Motortreibscheiben 134 ab. Die Steriladapterplatte 132 weist drehbare Kupplungsscheiben auf, die eine Übertragung von Rotationsbewegungen von der Antriebseinheit auf die Antriebsscheiben des chirurgischen Instruments 10 zulässt.

Die Steriladapterplatte 132 ist mit einem sterilen Überzug 136 verbunden. Der sterile Überzug 136 umgibt die Antriebseinheit 122 und die Führungseinrichtung 126 zumindest teilweise. Der sterile Überzug 136 kann aus einer Folie sein, die mit der Antriebseinheit 122 gekoppelt ist. Die Antriebseinheit 122 weist eine in Form einer Nut ausgebildete Ausnehmung 138 auf, durch die sich das der Schaft des chirurgische Instruments 10 erstrecken kann, wenn das chirurgische Instrument 10 mit der Antriebseinheit 122 von proximal aus gekoppelt ist. Dies bedeutet, dass das Gehäuse 12 des chirurgischen Instruments 10 sich an der Schnittstelle 124 der Antriebseinheit 122 befindet, sich der Schaft des chirurgischen Instruments 10 durch die Ausnehmung 138 in Form einer Nut und durch die Führungsöffnung 130 erstreckt. Die Ausnehmung 138 kann sich in Längsrichtung der Antriebseinheit 122 erstrecken.

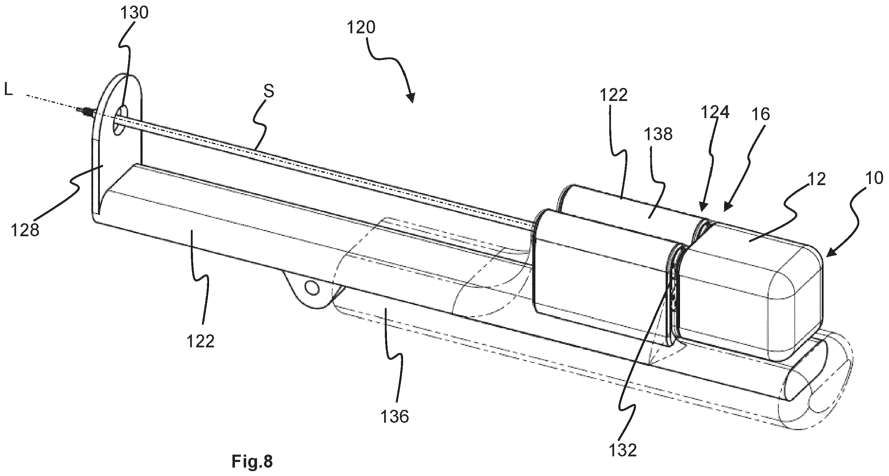

Figur 8 zeigt eine perspektivische Ansicht des Antriebssystems 120. Das chirurgische Instrument 10 wurde von proximal aus mit der Antriebseinheit 122 gekoppelt. Zwischen den Schnittstellen 124 und 16 der Antriebseinheit 122 und des chirurgischen Instruments 10 befindet sich die Steriladapterplatte 132. Der Schaft S des chirurgischen Instruments 10 erstreckt sich durch die Ausnehmung 138 der Antriebseinheit 122 und durch die Führungsöffnung 130. Das chirurgische Instrument 10 kann in Richtung der Längsachse L von proximal aus mit der Antriebseinheit 122 gekoppelt werden. Das chirurgische Instrument 10 bzw. das Gehäuse 12 des chirurgischen Instruments 10 befindet sich somit an dem von dem Führungsvorsprung 128 abgewandten Ende der Antriebseinheit 122.

In den Figuren 7 und 8 wird deutlich, dass zunächst die Steriladapterplatte 132 an der Schnittstelle 124 der Antriebseinheit 122 angebracht wird. Falls der sterile Überzug 136 mit der Steriladapterplatte 132 verbunden ist, kann dieser die Antriebseinheit 122 und die Führungseinrichtung 126 zumindest abschnittsweise umgeben. Im Anschluss an diesen Schritt wird das chirurgische Instrument 10 mit seinem Gehäuse 12 und dem darin angeordneten Lenkgetriebe 18 von proximal aus auf die Steriladapterplatte 132 gesteckt. Die Kopplung erfolgt insbesondere in Längsrichtung L des chirurgischen Instruments 10. Durch diese Fügerichtung in einer Linie mit einem Trokar (nicht gezeigt) kann das chirurgische Instrument 10 während einer Operation in einer durchgehenden Bewegung weiter nach distal in den Patienten hineingeschoben werden.

Die vorliegende Erfindung betrifft ein chirurgisches Instrument 10 mit einem Gehäuse 12, das am proximalen Ende 14 des chirurgischen Instruments 10 angeordnet ist, einer an dem Gehäuse 12 angeordneten Schnittstelle 16 zur Kopplung mit einer Antriebseinheit 122, einem in dem Gehäuse 12 angeordneten Lenkgetriebe 18, wobei das Lenkgetriebe 18 eine räumlich ausrichtbare Taumelscheibe 20 aufweist, und wenigstens einem Spindeltrieb 22, 24 zum Antreiben 22, 24 des Lenkgetriebes 18. Die vorliegende Erfindung betrifft ferner ein steriles Antriebssystem 120 für ein solches chirurgisches Instrument 10 und ein Verfahren für die sterile Montage eines Antriebssystems 120. Die Zeichnungen, die Beschreibung und die Ansprüche enthalten zahlreiche Merkmale in Kombination. Es versteht sich, dass die vorstehend genannten Merkmale nicht nur in der jeweils angegebenen Kombination, sondern auch in anderen Kombinationen oder in Alleinstellung verwendbar sind, ohne den Rahmen der vorliegenden Erfindung zu verlassen.

### Bezugszeichenliste

- 10: Chirurgisches Instrument
- 12: Gehäuse
- 14: proximales Ende
- 16: Schnittstelle
- 18: Lenkgetriebe
- 20: Taumelscheibe
- 22: Erster Spindeltrieb
- 24: Zweiter Spindeltrieb
- 26: Kopplungsfläche
- 28,30: Antriebsscheibe
- 32: ersten Schieber
- 34: zweiter Schieber
- 36: Klammer
- 38: Kugelkopf
- 40,42: Kugelpfannen
- 44: Klammerbasis
- 46,48: Klammerarme
- 50,52: Endabschnitte
- 54: Klammeröffnung
- 56: Nut
- 60: Axiale Kontaktflächen der Nut 58
- 62: Lenkring
- 68,70,72,74: Kegelräder
- 76,78: axiale Kontaktflächen der Klammer 36
- 80: Innenfläche der Klammer
- 82,84: Antriebsscheiben
- 88: Stirnrad
- 90: Zahnradgetriebe
- 92: Antriebsspindel
- 94: Antriebsscheibe
- 96: Lenkdrähte
- 98: Schaftwelle
- 100: Kreuzgelenk
- 102: Zahnrad
- 104: Lagerwelle
- 106,108,112: Kegelverzahnungsabschnitte
- 120: Antriebssystem
- 122: Antriebseinheit
- 124: Schnittstelle
- 126: Führungseinrichtung
- 128: Führungsvorsprung
- 130: Führungsöffnung
- 132: Steriladapterplatte
- 134: Motortreibscheiben
- 136: steriler Überzug
- 138: Ausnehmung
- S: Schaft
- L: Längsachse
- D1: Drehachse
- D2: Drehachse
- D3: Drehachse
- D4: Drehachse

## Patentansprüche

1. Chirurgisches Instrument (10), umfassend:
ein Gehäuse (12), das am proximalen Ende (14) des chirurgischen Instruments (10) angeordnet ist,
eine an dem Gehäuse (12) angeordnete Schnittstelle (16) zur Kopplung mit einer Antriebseinheit (122),
ein in dem Gehäuse (12) angeordnetes Lenkgetriebe (18), wobei das Lenkgetriebe (18) eine räumlich ausrichtbare Taumelscheibe (20) aufweist, und
wenigstens einen Spindeltrieb (22, 24) zum Antreiben (22, 24) des Lenkgetriebes (18).

2. Chirurgisches Instrument (10) nach Anspruch 1,
wobei die Schnittstelle (16) an einer senkrecht zur Längsachse (L) eines Schafts (S) verlaufenden Kopplungsfläche (26) des Gehäuses (12) angeordnet ist, wobei der Schaft (S) durch die Kopplungsfläche (26) verläuft.

3. Chirurgisches Instrument (10) nach Anspruch 1 oder 2,
wobei das chirurgische Instrument (10) zumindest einen ersten Spindeltrieb (22) und einen zweiten Spindeltrieb (24) aufweist, deren Drehachsen (D1, D2) parallel zueinander und parallel zur Längsachse (L) des chirurgischen Instruments (10) verlaufen.

4. Chirurgisches Instrument (10) nach Anspruch 3,
wobei der erste Spindeltrieb (22) und der zweite Spindeltrieb (24) jeweils eine Antriebsscheibe (28, 30) aufweisen, die an der Kopplungsfläche (26) angeordnet sind.

5. Chirurgisches Instrument (10) nach einem der Ansprüche 1 bis 4,
wobei das Lenkgetriebe (18) einen ersten Schieber (32) und einen zweiten Schieber (34) aufweist,
wobei der erste Schieber (32) dem ersten Spindeltrieb (22) und der zweite Schieber (34) dem zweiten Spindeltrieb (24) zugeordnet ist.

6. Chirurgisches Instrument (10) nach Anspruch 5,
wobei das Lenkgetriebe (18) eine Klammer (36) aufweist, die an der Taumelscheibe (20) angeordnet und mit dem ersten Schieber (32) und dem zweiten Schieber (34) gekoppelt ist.

7. Chirurgisches Instrument (10) nach Anspruch 6,
wobei der erste Schieber (32) und der zweite Schieber (34) jeweils einen Kugelkopf (38) aufweisen, die in Kugelpfannen (40, 42) an der Klammer (36) eingreifen.

8. Chirurgisches Instrument (10) nach Anspruch 6 oder 7,
wobei die Klammer (36) eine Klammerbasis (44) und zwei Klammerarme (46, 48) aufweist, wobei die Kugelpfannen (40, 42) an Endabschnitten (50, 52) der Klammerarme (46, 48) angeordnet sind.

9. Chirurgisches Instrument (10) nach Anspruch 8,
wobei die Klammerarme (46, 48) zwischen ihren Endabschnitten (50, 52) eine Öffnung (54) der Klammer (36) festlegen, wobei die Öffnung (54) der Klammerbasis (44) gegenüberliegt.

10. Chirurgisches Instrument (10) nach einem der Ansprüche 6 bis 9,
wobei die Klammer (36) zumindest abschnittsweise elastisch ausgebildet ist.

11. Chirurgisches Instrument (10) nach einem der Ansprüche 8 bis 10,
wobei die Elastizität der Klammer (36) zumindest über die geometrische Dicke und/oder die Materialwahl der Klammerbasis (44) einstellbar ist.

12. Chirurgisches Instrument (10) nach einem der Ansprüche 6 bis 11,
wobei die Taumelscheibe (20) eine Nut (56) aufweist, die einander gegenüberliegende und zueinander parallel verlaufende axiale Kontaktflächen (58, 60) für die Klammer (36) aufweist, wobei die Klammer (36) in der Nut (56) aufgenommen ist.

13. Chirurgisches Instrument (10) nach einem der Ansprüche 6 bis 12,
wobei die Klammer (36) mehr als 180°, insbesondere einen Winkelbereich zwischen 270°und 350° des Umfangs der Taumelscheibe (36) umgibt.

14. Chirurgisches Instrument (10) nach einem der Ansprüche 1 bis 5,
wobei das Lenkgetriebe (18) einen mit der Taumelscheibe (20) gekoppelten Lenkring (62) aufweist.

15. Chirurgisches Instrument nach Anspruch 14,
wobei der erste Spindeltrieb (22) und der zweite Spindeltrieb (24) mit Zahnrädern (64, 66) zum Antreiben von zumindest zwei Kegelräder (68, 70, 72, 74) zusammenwirken, die mit dem Lenkring (62) gekoppelt sind.

16. Antriebssystem (120) für ein chirurgisches Instrument (10),
wobei das Antriebsystem (120) eine an einem Roboterarm anbringbare Antriebseinheit (122), einer an der Antriebseinheit (122) angebrachten Steriladapterplatte (132), der eine Schnittstelle (124) an der Antriebseinheit (124) zumindest teilweise abdeckt, und das chirurgische Instrument (10) nach einem der Ansprüche 1 bis 15 umfasst, wobei das chirurgische Instrument (10) über die Schnittstelle (16) mit der Antriebseinheit (122) von proximal aus gekoppelt ist.

17. Verfahren für die sterile Montage eines Antriebssystems (120) für ein chirurgisches Instrument (10), wobei das Antriebsystem (120) eine an einem Roboterarm anbringbare Antriebseinheit (122), und das chirurgische Instrument (10) nach einem der Ansprüche 1 bis 15 umfasst, wobei das Verfahren die folgenden Schritte umfasst:
Anbringen einer Steriladapterplatte (132) an der Antriebseinheit (122), der eine Schnittstelle (124) an der Antriebseinheit (122) zumindest teilweise abdeckt, und
Anbringen des chirurgischen Instruments (10) an der Schnittstelle (124) der Antriebseinheit (122) von proximal aus.
